# EUROPEAN PATENT APPLICATION

(11) **EP 3 795 589 A1**
(43) Date of publication of application: **24.03.2021**
(21) Application number: 20174403.4
(22) Date of filing: 09.06.2017
(51) Int. Cl.: C07K 14/81

(54) **PEPTIDES FOR THE INHIBITION OF TRYPSIN**

(30) Priority: 10.06.2016 NO 20160995; 30.12.2016 NO 20162073; 11.05.2017 NO 20170778
(62) Divisional of application: 17737652.2
(71) Applicant: Aqua Health AS, 5252 Søreidgrend (NO)
(72) Inventor: McKinley, Scott, West Vancouver British Columbia V7V 1N6 (CA)
(74) Representative: Acapo AS

(57) **Abstract**

Peptides, which inhibit trypsin, are described. Also described are pharmaceutical compositions comprising such peptides, and the use of such peptides and pharmaceutical compositions for the inhibition of trypsin enzymes.

## Description

### Field of the invention

The present invention relates to peptides and pharmaceutical compositions comprising such peptides for the inhibition of trypsin. The peptides can be used in pharmaceutical composition for inhibiting an attachment of a parasite to a host, e.g. for inhibiting the infestation and infection of a caligid copepod on a salmonid, or for the prevention and/or treatment of infections in salmonids caused by sea lice.

### Background of the invention

Sea lice present a large economic burden for fish farmers. Sea lice are obligate ectoparasitic copepods on the external surface of marine fish. The term sea lice commonly refer to *Lepeophtheirus salmonis* and *Caligus rogercresseyi.*

*L. salmonis* affects inter alia wild and farmed salmon and the rainbow trout industry in Scotland, Ireland, Norway, Faeroe Islands, the northern Atlantic and Pacific coasts of Canada and the U.S., and the Pacific coast of Japan.

The life cycle of *L. salmonis* consists of 10 different stages and lasts 7-8 weeks at 10°C. Naupilus and copepodid are free swimming and non-parasitic stages, and chalimus, pre adult and adult lice are attached and parasitic stages.

A characteristic feature of attachment and feeding sites of caligid copepods on many of their hosts is that a trypsin-like activity is secreted by *L. salmonis* onto the salmon skin. It is believed that it is used by the sea lice to feed on the salmon mucus, skin and blood and to protect the sea lice from the salmon immune response.

The salmon louse, *L. salmonis,* is known to secrete a number of proteases upon parasitic infection of its host *Salmo salar.* A trypsin-like serine protease appears to be the major protease secreted by the louse. This enzyme may provide a potential drug target for treatment of *L. salmonis* infection in farmed salmon. Based on the modelled 3D structure of the *L. salmonis* trypsin-1 protein (LsTryp1), 11 peptides have been designed with an amino acid sequence that should allow specific binding to LsTryp1 and inhibition of its protease activity. Here we test the LsTryp1 activity of these peptides against recombinant LsTryp1 that has been expressed in *E. coli.*

### Summary of the invention

A first aspect of the present invention relates to a peptide, wherein said peptide inhibits a trypsin protease protein of a Caligidae, wherein the peptide comprises an amino acid sequence selected from the group consisting of SEQ ID NO 4, SEQ ID NO 6, SEQ ID NO 7, SEQ ID NO 8 or SEQ ID NO 11 and variants thereof being at least 70% identical over the entire sequence with any of the sequences SEQ ID NO 4, SEQ ID NO 6, SEQ ID NO 7, SEQ ID NO 8 or SEQ ID NO 11.

In a preferred embodiment inhibits said peptide a trypsin protease protein, wherein the peptide comprises an amino acid sequence selected from the group consisting SEQ ID NO 4, SEQ ID NO 65, SEQ ID NO 7, SEQ ID NO 8 or SEQ ID NO 11.

In a preferred embodiment comprises the peptide the amino acids of SEQ ID NO 4.

In a preferred embodiment comprises the peptide the amino acids of SEQ ID NO 6.

In a preferred embodiment comprises the peptide the amino acids of SEQ ID NO 7.

In a preferred embodiment comprises the peptide the amino acids of SEQ ID NO 8.

In a preferred embodiment comprises the peptide the amino acids of SEQ ID NO 10.

In a preferred embodiment inhibits the peptide a trypsin protease from a parasite.

In a preferred embodiment is the trypsin protease from an ectoparasite.

In a preferred embodiment inhibits said peptide inhibits a trypsin protease protein from a Caligidae.

In a preferred embodiment is the Caligidae selected from the group consisting of Pseudocaligus, Caligus and Lepeophtheirus.

In a preferred embodiment is said Caligidae Lepeophtheirus salmonis.

I a preferred embodiment is said trypsin protease LsTryp1 or rLsTryp1.

A second aspect of the present invention relates to a pharmaceutical composition for use in the prevention and/or treatment of a disease or infection by inhibiting the activity of a trypsin proteinase, wherein said pharmaceutical composition comprises a peptide which inhibits a trypsin protease protein, wherein the peptide comprises an amino acid sequence selected from the group consisting of SEQ ID NO 4, SEQ ID NO 6, SEQ ID NO 7, SEQ ID NO 8 or SEQ ID NO, and variants thereof being at least 70% identical over the entire sequence with any of the sequences SEQ ID NO 4, SEQ ID NO 6, SEQ ID NO 7, SEQ ID NO 8 or SEQ ID NO.

In a preferred embodiment is the disease or infection caused by a parasite, and wherein the disease or infection is prevented or treated by inhibiting the activity of a trypsin proteinase.

In a preferred embodiment inhibits said peptide a trypsin protease protein from a Caligidae.

I a preferred embodiment is said Caligidae selected from the group consisting of Pseudocaligus, Caligus and Lepeophtheirus.

In a preferred embodiment is said Caligidae salmon louse Lepeophtheirus salmonis.

In a preferred embodiment is said trypsin protease LsTryp1 or rLsTryp1.
wherein said peptide inhibits a trypsin protease protein, wherein the peptide comprises an amino acid sequence selected from the group consisting of SEQ ID NO 3, SEQ ID NO 5 or SEQ ID NO 10 and variants thereof being at least 70% identical over the entire sequence with any of the sequences SEQ ID NO 3, SEQ ID NO 5 or SEQ ID NO 10.

In a preferred embodiment inhibits said peptide a trypsin protease protein, wherein the peptide comprises an amino acid sequence selected from the group consisting of SEQ ID NO 3, SEQ ID NO 5 or SEQ ID NO 10.

In a preferred embodiment comprises the peptide the amino acids of SEQ ID NO 3.

In a preferred embodiment comprises the peptide the amino acids of SEQ ID NO 5.

In a preferred embodiment comprises the peptide the amino acids of SEQ ID NO 10.

In a preferred embodiment inhibits the peptide a trypsin protease from a parasite.

In a preferred embodiment is said trypsin protease from an ectoparasite.

In a preferred embodiment inhibits said peptide a trypsin protease protein from a Caligidae.

In a preferred embodiment is said Caligidae selected from the group consisting of Pseudocaligus, Caligus and Lepeophtheirus.

In a preferred embodiment, said Caligidae is Lepeophtheirus salmonis.

In a preferred embodiment is said trypsin the *L. salmonis* trypsin-1 protein.

A second aspect of the present invention relates to a pharmaceutical composition for use in the prevention and/or treatment of a disease or infection by inhibiting the activity of a trypsin proteinase, wherein said pharmaceutical composition comprises a peptide which inhibits a trypsin protease protein, wherein the peptide comprises an amino acid sequence selected from the group consisting of SEQ ID NO 3, SEQ ID NO 5 or SEQ ID NO 10, and variants thereof being at least 70% identical over the entire sequence with any of the sequences SEQ ID NO 3, SEQ ID NO 5 or SEQ ID NO 10.

In a preferred embodiment is the disease or infection caused by a parasite, and the disease or infection is prevented or treated by inhibiting the activity of a trypsin proteinase.

In a preferred embodiment inhibits said peptide a trypsin protease protein from a Caligidae.

In a preferred embodiment is said Caligidae selected from the group consisting of Pseudocaligus, Caligus and Lepeophtheirus.

In a preferred embodiment is said Caligidae the salmon louse Lepeophtheirus salmonis.

### Description of the drawings

Figure 1 shows a plasmid map of pET40-LsTryp1.
Figure 2 shows a portion of the sequencing data for LsTryp1 aligned to the expected sequence of pET40-LsTryp1. It shows the P159A mutation, which was the only amino acid change from expected based on published genomic L. salmonis sequence.

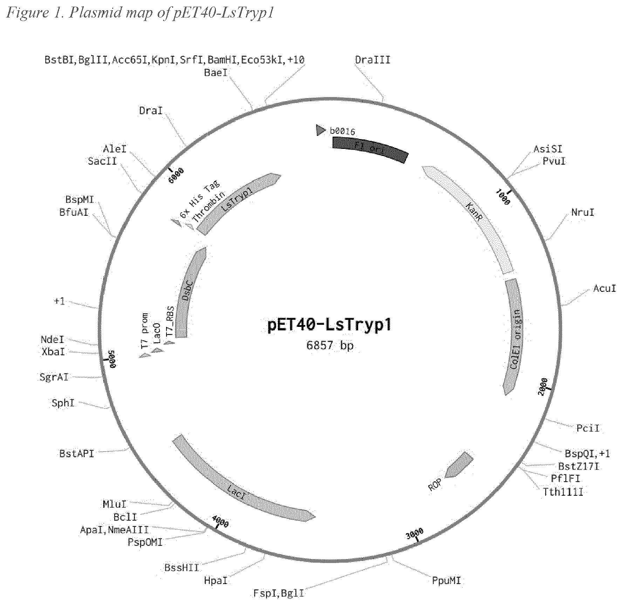

Figure 1 shows a Western blot for His-tag proteins isolated from BL21 with pET40-LsTryp1 (lane 1), pET-40b lacking the LsTryp1 gene (lane 2), pET40-LsTryp1 without induction of protein expression (lane 3) and protein size ladder (Fermentas; lane 4).

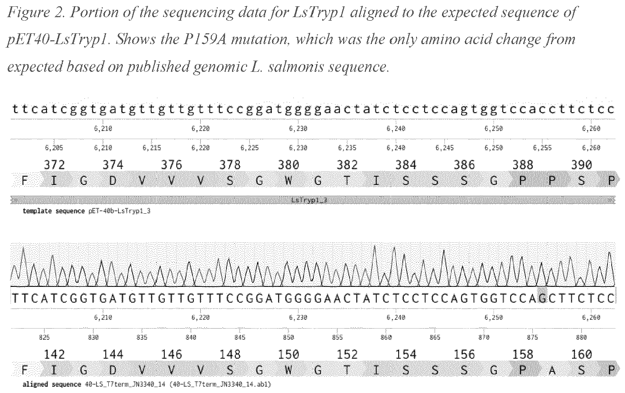

Figure 2 shows the analysis of LsTryp1 purification. A) Coomassie stained SDS-PAGE gel. B) Western blot for His-tagged proteins (same samples as in "A"). Lane 1: flow through of IMAC column, Lanes 2-4: Sequential elution from IMAC column, Lane 5: Protein ladder (NEB)

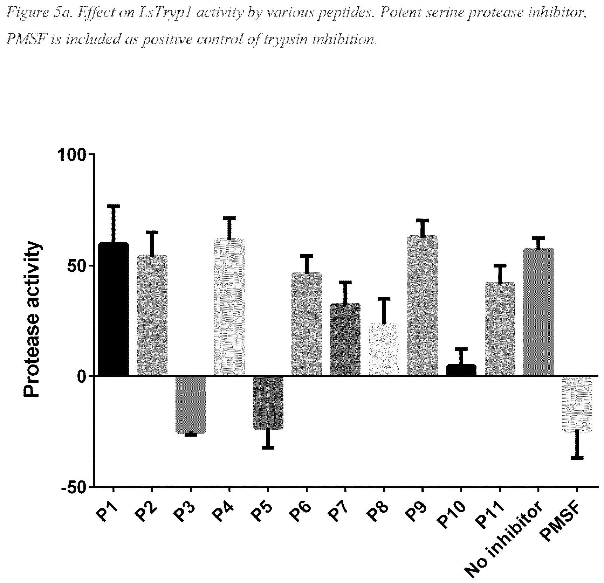

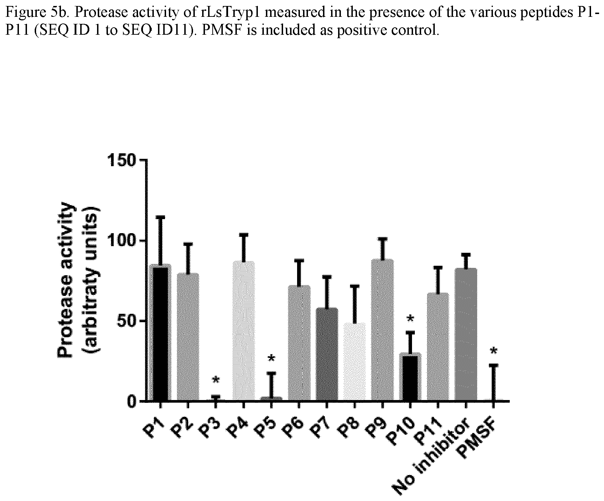

Figure 5a and 5b shows the effect on LsTryp1 and rLsTryp1 activity by various peptides. Potent serine protease inhibitor, PMSF is included as positive control of trypsin inhibition.

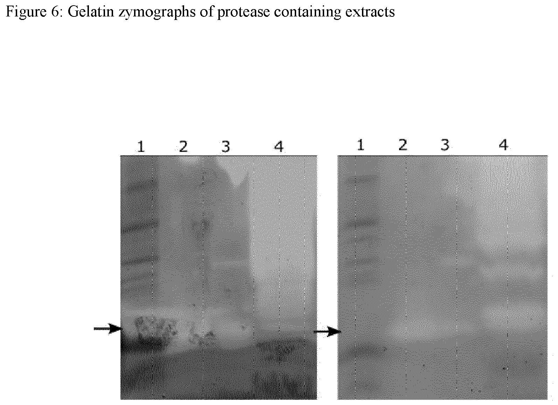

Figure 6 shows gelatin zymograph of protease containing extracts from various sources. A) No inhibitor present. B) In the presence of a mixture of SEQ ID NO 3 (P3) and SEQ ID NO 5 (P5) at a concentration of 50µg/mL each.

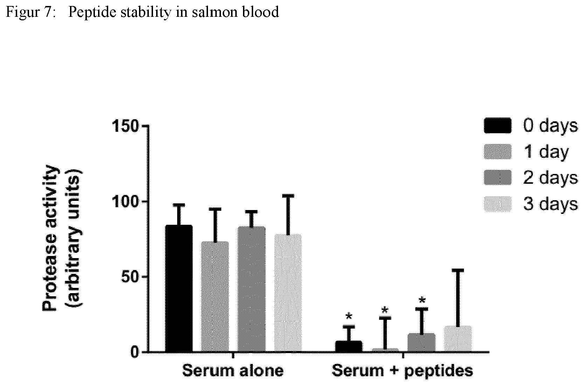

Figure 7 shows the protease activity of rLsTrp after incubation in S. salar serum over 3 days as a measure of peptide stability in salmon blood. *denotes a statistically significant difference compared to serum without peptide collected at the same time point (p < 0:05).

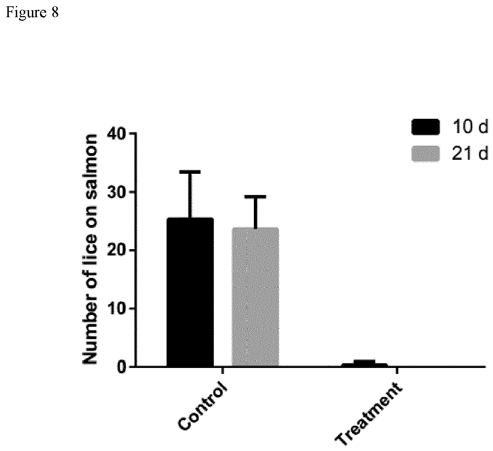

Figure 8 shows a graph of a number of lice found on control vs. peptide treated salmon from in vivo experiment 1 (high peptide concentration). Observations made 10 days and 21 days post lice exposure. Error bars represent standard deviation.

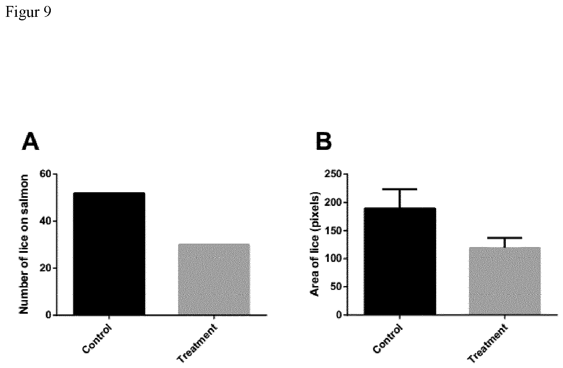

Figure 9 shows a graph of a number of lice found and area of those lice on control vs. peptide treated salmon from in vivo experiment #2 (low peptide concentration). Observations made seven days post lice exposure and 17 days post administration of peptide. Error bars ('size' column only) represent standard error of the mean. * indicates a statistically significant difference (P < 0:01).

### Detailed description of the invention

We have prepared a number of different peptide sequences and tested their ability to inhibit a trypsin from sea lice.

### Experimental section

### Materials and methods

### Strains and culture conditions

*E. coli* DH10B was used for cloning and production of plasmid DNA. Cultures were grown in ZYM-505 medium at 37°. DNA manipulations were performed using standard procedures. Plasmid DNA was purified using QIAprep Spin Miniprep Kit (Qiagen). *E. coli* BL21(DE3)pLysS (referred to as BL21 from here on) was used as a protein expression host for LsTryp1. BL21 was routine culture of BL21 was in MDAG-135 and protein expression was performed in auto-induction medium, MDA-5052. Antibiotics kanamycin (Km) and chloramphenicol (Cm) were used at 100 and 33 µg/mL, where appropriate.

### Cloning, expression and purification ofLsTrypl

Approximately 15 adult *L. salmonis* were harvested from infected salmon and placed in 10 mL RNAlater reagent (Qiagen). Total RNA was purified using the RNeasy Mini Kit (Qiagen). The RNA preparation was cleared of any genomic DNA carry-over by DNaseI digestion (Fermentas) followed by heat inactivation of the DNaseI enzyme. First-strand cDNA synthesis was performed using 1 µg *L. salmonis* RNA and M-MulV reverse transcriptase (200 U) primed with an oligo-dT primer in the presence of RNase inhibitor, by manufacturer's instructions (NEB). PCR amplification of the *LsTryp1* gene was performed with PrimeSTAR GXL DNA Polymerase (Clonetech) using primers F-LsTryp1_3 (5'-gttccccctcagatcaaatactctgag-3') and R-LsTryp1_1 (5'-ctattggtgttcagcaatccagtcaatg-3'; IDT) with an annealing temperature of 60°C and 35 cycles. PCR product of the expected size was (672 bps) was confirmed by agarose gel electrophoresis and this product was purified by gel extraction using the NucleoSpin Gel and PCR Clean-up kit (MACHEREY-NAGEL GmbH & Co). PCR product of the LsTryp1 gene was ligated into pET-40b expression vector (Novagen) which had been digested with restriction enzyme, ScaI (NEB) using T4 DNA ligase (NEB). The product of this ligation was used to transform *E. coli* DH10B. Km resistant tranformants were screened by PCR for the presence of plasmid containing the LsTryp1 gene in the correct orientation. Plasmid DNA was extracted from a positive clone and sequence of the correct LsTryp1 gene was confirmed by DNA sequencing (Eurofins MWG Operon). This plasmid (pET40-LsTryp1) was introduced into *E. coli* BL21 for protein expression. 5 mL culture was grown for 24 h in auto-induction medium at 30°C. Cells were then collected by centrifugation and lysed in BugBuster Protein Extraction Reagent (Novagen) supplemented with benzonase and lysozyme and purified over a Ni-NTA His•Bind Resin (Novagen) column. Presence of LsTryp1 was monitored by SDS-PAGE gel electrophoresis and western blot, performed by standard methods. SDS-PAGE gels were stained with coomassie blue dye. For western blotting, protein from SDS-PAGE was transferred to nitrocellulose membranes and probed with rabbit α-His-tag antibody and IR800 conjugated α-rabbit IgG antibody (Rockland Immunochemicals Inc.) visualized on an Odyssey CLx IR scanner (LI-COR Biosciences).

### Protease inhibition assay

Protease activity of recombinant LsTryp1 protein was assayed using the Pierce Fluorescent Protease Assay Kit by manufactures instructions (Thermo-Fisher Scientific) in 384-well microtitre plate (White, low protein binding assay plate; Greiner Bio-One). Activity of LsTryp1 was measured in the presence or absence of each of the peptides of interest. Lyophilized peptides were reconstituted in assay buffer (10 mM tris, pH 7.4) to make stock solutions with a final concentration of 1 mM. Peptides were added to the FITC-casein substrate to a final concentration of 100 µM. Recombinant LsTryp extracted from *E. coli* BL21 was drop dialyzed on a 0.025 µm, mixed cellulose esters membrane filter against assay buffer for 2 hours then diluted 1:2 in assay buffer. 35 µL of this LsTryp1 solution was added to 35 µL of substrate with or without peptide for a final assay volume of 70 µL. Lysate extracted from of a culture of BL21 lacking the recombinant LsTryp1 gene, but otherwise processed in the same manor to the LsTryp1 extraction, was used as baseline to control for possible carryover of endogenous *E. coli* proteases. Serine protease inhibitor, phenylmethane-sulfonyl fluoride (PMSF) was included as a positive control for trypsin inhibition. All test samples were assayed in quadruplicate. Protease digestion was allowed to proceed for 30 minutes after LsTryp1 addition before reading output on a SpectraMax M plate reader (Molecular Devices) with an excitation wavelength of 494 nm and emission wavelength of 521 nm.

### Results

### Cloning of the LsTryp1 gene and expression of active LsTryp1

PCR of the LsTryp1 gene with the primer sequences presented above produced a DNA fragment of consistent with the size expected for the PCR using *L. salmonis* cDNA as a template (672 base pairs). The PCR amplified fragment includes only the enzyme domain and lacks the signal peptide and activation peptide of the natural full-length transcript to avoid problems of improper processing to mature protease in the *E. coli* host.

Insertion of the LsTryp1 PCR product into the ScaI site of pET-40b results in an in-frame translational fusion of LsTryp1 to the upstream DsbC gene, separated by a 6x histidine tag (His-tag) and a thrombin cleavage site. The DsbC gene encodes a periplasm localized disulfide bond isomerase enzyme, which, fused to the LsTryp1 protein, should cause it to be excreted to the periplasmic space of *E. coli.* The periplasm provides a more favorable environment for the proper folding of disulfide bond-rich proteins (there are four disulfide bonds in LsTryp1) as does the enzyme activity of the DsbC fusion partner. The His-tag allows the protein to be purified by immobilized metal ion affinity chromatography (IMAC). A map of the plasmid is shown in Figure 1, with important elements annotated.

The Dsb-LsTryp1 fusion gene in pET40-LsTryp1 is downstream of a T7 polymerase promoter and relies on the presence of the T7 RNA polymerase protein to be present in the host cell for transcription. The BL21(DE3)pLysS expression host contains the gene for T7 RNA polymerase, but this gene is under control of the Lac repression (LacI), which is in a repressed state unless lactose is present and glucose is not. This allows controlled expression of our protease until which time these conditions are met. To limit potential toxic effects of LsTryp1 on the host cells, expression is delayed until late in the growth phase. This was achieved through the use of auto-induction medium, which contains both glucose and lactose in a specific ratio so that glucose is present and used as the main energy source while the culture is growing to sufficient density. At the point at which glucose (which is used by the bacteria preferentially to lactose) is used up by the host cells, lactose repression of the T7 RNA polymerase is lifted and high-level expression of the DsbC-LsTrypl fusion begins. After a culture period of a length appropriate to allow for induction of T7 RNA polymerase expression and accumulation of the recombinant fusion protein, cells were harvested and a soluble protein extract was isolated.

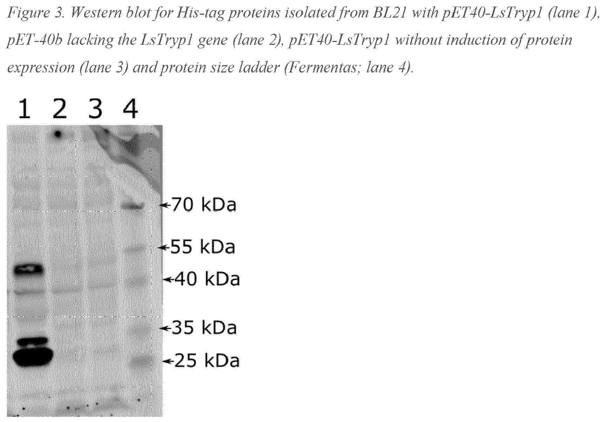

Protein extracts were analyzed for presence of the DsbC-LsTryp1 fusion by western immune blot probed with an antibody specific to the 6x His tag epitope located in the linker region between the DsbC and LsTryp1. Figure 3 shows multiple reactive bands corresponding the expected sizes of the fusion protein and trypsin cleavage products of the full-length fusion protein. The full-length product is expected to be a protein of 49.2 kDa. The linker region between DsbC and LsTryp 1 contains a trypsin cleavage site just a few amino acids up from the start of LsTryp1. Cleave by a protease with trypsin-like specificity would result in a 25.4 kDa fragment composed of DsbC and the 6x His-tag. The blot in Figure 3 clearly shows protein bands of these approximate sizes. A 27.1 kD fragment corresponding to the LsTryp1 protein should also be produced, but this would not be expected to react with the □-His-tag antibody. A third band observed likely corresponds to a second trypsin cleavage fragment. These results show that the DsbC-LsTrypl is expressed in the *E. coli* BL21. The presence of smaller fragments of the full-length protein of a size congruent with trypsin cleavage at an exposed trypsin cleavage site is consistent with expression of an active trypsin protease. However, it is possible that the cleavage observed is due to activity of endogenous periplasmic proteases, such as the trypsin-like serine protease DegP. Major involvement of DegP in cleavage of the DsbC-LsTrypl fusion is unlikely as DegP is a heat shock protein and is not highly expressed at 30°, the temperature at which these cells were cultured.

### Purification ofLsTrypl

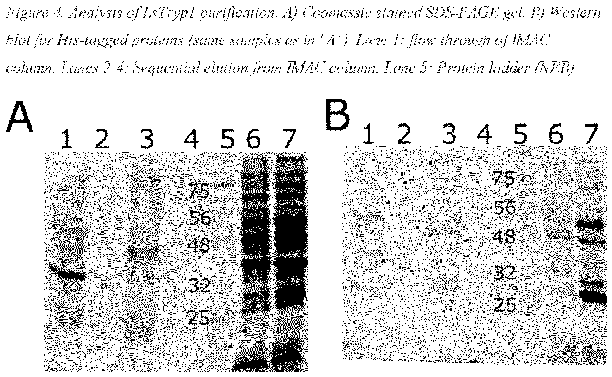

The presence of the 6x His-tag motif in the linker region between LsTryp1 and its fusion expression partner, DsbC, could allow purification of the full-length fusion protein through specific binding to a IMAC resin. However, as this fusion protein was found to have already been processed into its cleaved state, releasing the LsTryp1 portion from the 6x His affinity tag, purification over a Ni-NTA resin will likely result in major loss of the recombinant LsTrypsin enzyme. Such a purification was attempted anyway to verify this conclusion. When the soluble total protein fraction extracted from BL21 was subjected to IMAC purification, a band corresponding to the size of the free LsTryp1 cleavage product (27.1 kDa) did not bind to the resin, but was instead found in the column flow through (Figure 4A, lane 1), and not in significant amount in any of the fractions eluted from the column with imidazole. Western blots of these same fractions with an α-His tag antibody did strongly detect the presence of a the His-tag epitope in the flow-through fraction (Figure 4B), further supporting the conclusion that the 27.1 kDa band in this fraction is indeed recombinant LsTryp1.

Despite the finding that IMAC purification was not successful due to cleavage of LsTryp 1 from the affinity tag before the chromatography step, the protein lysate flow through from the IMAC column was greatly enriched for the LsTryp1 band (Figure 4). This is likely due to trypsin cleavage of contaminating proteins during the IMAC process. The large excess of other soluble protein from the bacterial lysates (Figure 4A, lane 5) is reduced to the point that the LsTryp1 band is the major protein component after the lysate is subjected to the IMAC process, despite no specific purification of LsTryp1 having occurred. This observation again further strengthens the conclusion of the successful recombinant production of an active protease. Furthermore, the apparent digestion of the majority of contaminating host proteins by the protease activity of recombinant LsTryp1 provides a fortuitous purification method: auto-purification by protease digestion of host proteins.

### Assay for inhibition by putative LsTryp1 inhibiting peptides

Protease activity of rLsTrp1 protein was assayed using the Pierce Fluorescent Protease Assay Kit by manufactures instructions (Thermo-Fisher Scientic) in a white, low protein binding, 384-well microtitre assay plate (Greiner Bio-One). Activity of rLsTrp1 was measured in the presence or absence of each of the 11 peptides of interest. Lyophilized peptides were reconstituted in assay buffer (10 mM tris, pH 7.4) to make stock solutions with a final concentration of 1 mM. Peptides were added to the FITC-casein substrate to a final concentration of 100 µM. rLsTrp extracted from E.coli BL21 was drop dialyzed on a 0.025 µm, mixed cellulose esters membrane filter against assay buffer for 2 h then diluted 1:2 in assay buffer. 35 µL of this LsTrp1 solution was added to 35 µL of substrate with or without peptide for a final assay volume of 70 µL. All test samples were assayed in quadruplicate. Protease digestion was allowed to proceed for 30 minutes after rLsTrp1 addition before reading output on a SpectraMax M plate reader (Molecular Devices) with an excitation wavelength of 494 nm and emission wavelength of 521 nm.

Protease activity of recombinant LsTryp1 was measured in the presence of 11 potential inhibitory peptides as given in table 1. Two of the 11 peptides (SEQ ID NO 3 (P3) and SEQ ID NO 5 (P5)) displayed an inhibitory activity indistinguishable in magnitude from that of the potent serine protease inhibitor, PMSF, at the same molar concentration. These data (figure 5) suggests that these two peptides are highly potent inhibitors of LsTryp1 activity, with activity comparable to small molecule inhibitors, but with the potential for much great target specificity. Proteases, such as LsTryp1, are important effectors of *L. salmonis* infection of salmon.

**Table 1**

| | | |
|---|---|---|
| Amino acid sequences for the peptides tested for inhibiting of LsTryp 1 | | |
| | | |
| SEQ ID NO 1 | P1 | VPEPVVQSPITTTT |
| SEQ ID NO 2 | P2 | VRCGRGRMLLLIERGQRFAT |
| SEQ ID NO 3 | P3 | VLGEEWHMEGCM |
| SEQ ID NO 4 | P4 | LMSFRKDAVMAIM |
| SEQ ID NO 5 | P5 | KLTINDASLC |
| SEQ ID NO 6 | P6 | TTTLLLHFTFDFNRVGV |
| SEQ ID NO 7 | P7 | DVVQVGYQMDHII |
| SEQ ID NO 8 | P8 | LNELETEMV |
| SEQ ID NO 9 | P9 | LKFMLPRRWCTGC |
| SEQ ID NO 10 | P10 | KFLHLDFNNGL |
| SEQ ID NO 11 | P11 | TPYYTTVNPTSPYTYDV |

### Effect of LsTrp inhibitory peptides on trypsins of salmon, lobster and mammalian origin

Zymography, a technique to detect activity of individual proteases in a complex sample, was used to determine the effect of the SEQ ID NO 3 (P3) and SEQ ID NO 5 (P5) mixture on trypsins for other organisms. A lysate of the E. coli strain used to express rLsTrp was used as the LsTrp source, and homogenates of intestines from S. salar and lobster were used as samples containing trypsin from these organisms. Purified bovine trypsin was available as a source of mammalian trypsin. Zymography analysis was done by standard procedures. Proteins of these samples were separated by SDS-PAGE under nonreducing conditions and without first boiling the samples in gels co-polymerized with 0.1% gelatin, which is used as the protease substrate. After electrophoresis SDS was removed from gels so that proteins could renature into their active state by incubation in renaturing buffer in the presence and absence of a peptide mixture of SEQ ID NO 3 (P3) and SEQ ID NO 5 (P5) at 100 µmol each for 2 hours. The gels were then fixed stained and destined as described previously for SDS-PAGE. Due to the gelatin present in the gel, the entire gel will stain positive for protein, except for regions were the gelatin has been removed through the action of proteases present within the gel. The zymograph shown in Figure 6 shows light bands of protein clearing in the lane corresponding to rLsTrp in the control gel not exposed peptide, but this clearing was absent in the gel incubated with the inhibitory peptide mixture. The other samples containing proteases from other organisms all showed bands of clearing despite the presence of SEQ ID NO 3 (P3) and SEQ ID NO 5 (P5), suggesting that these peptides do not display inhibitory activity against these proteases.

### Stability of LsTrp inhibitory peptides in salmon serum

The stability of peptides SEQ ID NO 3 (P3) and SEQ ID NO 5 (P5) in salmon blood was indirectly assayed by incubating the peptides in the serum fraction of freshly collected salmon blood for various times up to 3 days then analysing the protease activity of rLsTrp in the presence of serum plus peptide mixture. Peptides were reconstituted fresh from lyophilized aliquots at 10 mg/mL in 10 mM tris, pH 7.4 an added to the serum fraction of fresh salmon blood to a final concentration of 1 mg/mL each. Serum needed to be diluted 1:1 with the same tris solution used to reconstitute the peptides to avoid precipitation of serum proteins upon addition of peptides. The mixture of serum and peptides was incubated at room temperature with samples removed immediately after addition of peptide then again 1 day, 2 day and 3 days after addition of peptides. Removed portion was passed through a spin column membrane filtration device with a molecular weight cut-off of 10 kDa, which would allow the peptides to pass through (MW of 2.3 and 1.6 kDa for SEQ ID NO 3 (P3) and SEQ ID NO 5 (P5), respectively, while retaining proteins and larger peptides from the serum which should remove any serum peptidases. Salmon serum without peptides added (but also diluted in 10 mM tris and treated identically all other ways) was incubated and collected alongside the peptide and serum mixture, for use as a negative control. Samples were stored at -80°C until the protease assay was performed. Protease assay was performed as described above. Protease activity of rLsTrp in the presence of peptides mixed with salmon serum still show strong inhibitory activity when collected immediately after combining with the serum. Peptides that had been incubated in salmon serum for 1, 2 and 3 days showed a reduced ability to inhibit protease activity of the rLsTrp enzyme at the longer time points. This suggests that the peptides are present in their active form in the serum, but begin to loose potency (likely due to degradation by serum proteases) over a time range relevant to the 3 day period observed here. The fact that the a reduction in activity could still be measured going out three days for the peptides in fresh serum is a strong indication that they possess sufficient stability for in vivo activity to be possible. The results are shown in figure 7.

### Effects of the various peptides in protecting live salmon from sea lice infection

### High peptide concentration

Salmon of the treatment group were anesthetized in 50 mg/L tricaine methanesulfonate (TMS) were injected with a mixture of 100 mg each peptide P3 (SEQ ID NO3) and P5 (SEQ ID NO 5) suspended in 0.1 mL sesame oil. A control group of an equal number of salmon received sesame oil alone. Each group contained 12 fish for 24 in total. After a two hour period to allow recovery from the anesthetic, both treatment and control fish were infected with L. salmonis at 650 lice per fish in a single infection tank then and split into separate tanks after an hour of exposure to the copepodites. Three fish from each group were sacrificed and examined for lice presence on the gills and body after 3 days, 10 days and the remaining from each group were observed 21 days post lice exposure. At 3 days lice were very and accurate counts were not possible, so the first time point with usable data was 10 days. At the 10 day time point about two thirds of the lice were still present on the gills and few had matured to the stage at which they would be found on the body. At this time point and average of 24 lice were observed on the control fish, but only a single louse could be found on the peptide treated salmon combined. At the 21 day time point all fish of the treatment group were all healthy and completely free of lice. The control group, however, were thoroughly infested (Figure 8) and morbidly ill.

### Low peptide concentration

Fish were injected peritoneally with a mixture of 50 mg each of peptide P3 (SEQ ID NO 3) and P5 (SEQ ID NO 5) in 0.1 mL sesame oil (treatment group), with an equal number of fish receiving 0.1 mL sesame oil alone (control group). Ten days following administration of peptide fish were exposed to L. salmonis at 650 lice per fish. Seven days later fish were sacrificed by lethal dose of TMS immediately before observing fish for level of lice infestation.

As the fish were removed from the tanks it was noticed that the control fish were obviously more lethargic than fish from the peptide-treated group, as determined by a clear lack of fight upon transfer from the experimental tank. After sacrifice, salmon were photographed to document external physical injuries. Individuals from both groups displayed injuries consistent with L. salmonis infection, but severity of lesions found on control fish (C1-3) were striking in comparison to the treatment group (T1-3). Unfortunately, pictures taken to document fish in this experiment were not of sufficient quality to distinguish lice on the fish or other details such as damage to salmon surface. Fish C3 showed extensive damage to the base of the dorsal fin. Other individuals of both groups also exhibited lesions in this region, but not to the gruesome extent seen with C3. This same fish was barely responsive during transfer from the experimental tank, and appeared near death at the time of sacrifice. The treatment group individuals did not display significant injuries skin consistent with L. salmonis parasitism, but to an extent qualitatively less severe than that of the control group that did not receive the peptide treatment.

Lice were removed from the surface of each fish and counted. Many lice had become detached from the fish and could be recovered from the TMS solution in which the fish were sacrificed for both treatment groups. Control fish were associated with a total lice count of 52, compared to 30 for the treatment group (a 42.3% reduction; Figure 9).

### Definitions

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers.

The term "amino acid" refers to naturally occurring and synthetic amino acids, as well as amino acid analogs and amino acid mimetics that function in a manner similar to naturally occurring amino acids. Naturally occurring amino acids are those encoded by the genetic code, as well as those amino acids that are later modified, e.g., hydroxyproline, γ-carboxy-glutamate, and O-phosphoserine. Amino acid analogs refers to compounds that have the same basic chemical structure as a naturally occurring amino acid, i.e., an a carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Naturally encoded amino acids are the 20 common amino acids (alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine) as well as pyrrolysine, pyrroline- carboxy-lysine, and selenocysteine.

Percentage of sequence identity is determined by comparing two optimally aligned sequences over a comparison window, wherein the portion of the amino acid sequence or polynucleotide sequence in the comparison window may comprise additions or deletions (i.e. , gaps) as compared to the reference sequence (e.g., a polypeptide of the invention), which does not comprise additions or deletions, for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The term "variant" as used herein includes modifications, substitutions, additions, derivatives, analogs, fragments or chemical equivalents of the amino acid sequences disclosed herein that perform substantially the same function as the peptide inhibitors disclosed herein in substantially the same way.

Variants of the peptide inhibitors disclosed herein also include, without limitation, conservative amino acid substitutions. A "conservative amino acid substitution" as used herein, is one in which one amino acid residue is replaced with another amino acid residue without abolishing the desired function or activity of the peptide inhibitors disclosed herein. Conservative substitutions typically include substitutions within the following groups: glycine, alanine, valine, isoleucine, leucine, aspartic acid, glutamic acid, asparagine, glutamine, serine, threonine, lysine, arginine; and phenylalanine, tyrosine. Conserved amino acid substitutions involve replacing one or more amino acids of the polypeptides of the disclosure with amino acids of similar charge, size, and/or hydrophobicity characteristics. When only conserved substitutions are made the resulting variant should be functionally equivalent. Changes which result in production of a chemically equivalent or chemically similar amino acid sequence are included within the scope of the disclosure. If the peptide inhibitors of the present disclosure are made using recombinant DNA technology, variants of the peptide inhibitors may be made by using polypeptide engineering techniques such as site directed mutagenesis, which are well known in the art for substitution of amino acids. For example a hydrophobic residue, such as glycine can be substituted for another hydrophobic residue such as alanine. An alanine residue may be substituted with a more hydrophobic residue such as leucine, valine or isoleucine. A negatively charged amino acid such as aspartic acid may be substituted for glutamic acid. A positively charged amino acid such as lysine may be substituted for another positively charged amino acid such as arginine. The phrase "conservative substitution" also includes the use of a chemically derived residue in place of a non-derivatized residue provided that such polypeptide displays the requisite activity. In one embodiment, the peptides described herein comprise, consist essentially of, or consist of SEQ ID NO: 3, SEQ ID NO: 5, or SEQ ID NO: 10 and have 1,2 or 3 conservative amino acid substitutions. [0069] Variants of the peptide inhibitors of the present disclosure also include additions and deletions to the amino acid sequences disclosed herein.

A pharmaceutical composition comprises in addition to the peptide or peptide combination, therapeutically inactive ingredients, such as a pharmaceutically acceptable or physiologically acceptable excipient, carrier and/or adjuvants, which are well-known to the person skilled in the art and may include, but are not limited to, solvents, emulsifiers, wetting agents, plasticizers, solubilizers (e.g. solubility enhancing agents) coloring substances, fillers, preservatives, anti-oxidants, anti-microbial agents, viscosity adjusting agents, buffering agents, pH adjusting agents, isotonicity adjusting agents, mucoadhesive substances, and the like. Examples of formulation strategies are well-known to the person skilled in the art.

In some embodiments, the peptide may be formulated (e.g. mixed together) with immune-modifying agents like adjuvants. The adjuvant may be any conventional adjuvant, including but not limited to oxygen-containing metal salts, e.g. aluminium hydroxide, chitosan, heat-labile enterotoxin (LT), cholera toxin (CT), cholera toxin B subunit (CTB), polymerized liposomes, mutant toxins, e.g. LTK63 and LTR72, microcapsules, interleukins (e.g. IL-1 BETA, IL-2, IL-7, IL-12, INFGAMMA), G -CSF, MDF derivatives, CpG oligonucleotides, LPS, MPL, PL-derivatives, phosphophazenes, Adju-Phos(R), glucan, antigen formulation, liposomes, DDE, DHEA, DMPC, DMPG, DOC/Alum Complex, Freund's incomplete adjuvant, ISCOMs(R), LT Oral Adjuvant, muramyl dipeptide, monophosphoryl lipid A, muramyl peptide, and phospatidylethanolamine. Additional examples of adjuvants are described, for example, in "Vaccine Design- the subunit and adjuvant approach" (Edited by Powell, M. F. and Newman, M. J. ; 1995, Pharmaceutical Biotechnology (Plenum Press, New York and London, ISBN 0-306-44867-X) entitled "Compendium of vaccine adjuvants and excipients" by Powell, M. F. and Newman .

In some embodiments, the pharmaceutical composition may be formulated for parenteral administration, such as formulated for injection, e.g. subcutaneous and/or intradermal injection. Therefore, in some embodiments, the pharmaceutical composition may be a liquid (i.e. formulated as a liquid), including a solution, a suspension, a dispersion, and a gelled liquid. For example, a liquid pharmaceutical composition may be formed by dissolving a powder, granulate or lyophilizate of a peptide combination described herein in a suitable solvent and then administering to a subject. Suitable solvents may be any solvent having physiologically acceptable properties and able to dissolve the peptide combination in desired concentrations. A desired concentration may depend on the aliquot to be administered (i.e. to be injected) and the desired single dose.

A freeze-dried composition may also be formulated into a solid dosage form that is administered for example by the oral route such as by oral mucosa. Thus, in some embodiments, the pharmaceutical composition may be formulated for oral administration, for example for sublingual administration. Therefore, the pharmaceutical composition may be a solid dosage form, such as a freeze-dried solid dosage form, typically a tablet, a capsule or sachet, which optionally may be formulated for fast disintegration.

Pharmaceutical formulations and delivery systems appropriate for the compositions, methods and uses of the invention are known in the art (see, e.g., Remington : The Science and Practice of Pharmacy (2003) 20th ed., Mack Publishing Co., Easton, PA; Remington's Pharmaceutical Sciences (1990) 18th ed., Mack Publishing Co., Easton, PA; The Merck Index (1996) 12th ed., Merck Publishing Group, Whitehouse, NJ;

Pharmaceutical Principles of Solid Dosage Forms (1993), Technonic Publishing Co., Inc., Lancaster, Pa. ; Ansel ad Soklosa, Pharmaceutical Calculations (2001) 11th ed., Lippincott Williams & Wilkins, Baltimore, MD; and Poznansky et al., Drug Delivery Systems (1980), R. L. Juliano, ed., Oxford, N.Y., pp. 253-315).

As mentioned, pharmaceutical compositions can be formulated to be compatible with a particular route of administration, such as by intradermal or by sublingual administration. Thus, pharmaceutical compositions may include carriers, diluents, or excipients suitable for administration by various routes. Exemplary routes of administration for contact or in vivo delivery for which a composition can optionally be formulated include inhalation, intranasal, oral, buccal, sublingual, subcutaneous, intradermal, epicutaneous, rectal, transdermal, or intralymphatic.

For oral, buccal or sublingual administration, a composition may take the form of, for example, tablets or capsules, optionally formulated as fast-integrating tablets/capsules or slow-release tablets/capsules. In some embodiments, the tablet is freeze-dried, optionally a fast-disintegrating tablet or capsule suitable for being administered under the tongue.

The pharmaceutical composition may also be formulated into a "unit dosage form", which used herein refers to physically discrete units, wherein each unit contains a predetermined quantity of a peptide or peptide combination, optionally in association with a pharmaceutical carrier (excipient, diluent, vehicle or filling agent) which, when
administered in one or more doses, may produce a desired effect. Unit dosage forms also include, for example, ampules and vials, which may include a composition in a freeze-dried or lyophilized state (a lyophilizate) or a sterile liquid carrier, for example that can be added prior to administration or delivery in vivo. Unit dosage forms additionally include, for example, ampules and vials with liquid compositions disposed therein.

The application describes the following aspects of the invention:
1. A peptide, wherein said peptide inhibits a trypsin protease protein, wherein the peptide comprises an amino acid sequence selected from the group consisting of SEQ ID NO 3, SEQ ID NO 5 or SEQ ID NO 10 and variants thereof being at least 70% identical over the entire sequence with any of the sequences SEQ ID NO 3, SEQ ID NO 5 or SEQ ID NO 10.
2. A peptide, wherein said peptide inhibits a trypsin protease protein, wherein the peptide comprises an amino acid sequence selected from the group consisting of SEQ ID NO 3, SEQ ID NO 5 or SEQ ID NO 10.
3. A peptide in accordance with aspect 1 or 2, wherein the peptide comprises the amino acids of SEQ ID NO 3.
4. A peptide in accordance with aspect 1 or 2, wherein the peptide comprises the amino acids of SEQ ID NO 5.
5. A peptide in accordance with aspect 1 or 2, wherein the peptide comprises the amino acids of SEQ ID NO 10.
6. A peptide in accordance with aspect 1 or 2, wherein the peptide inhibits a trypsin protease from a parasite.
7. A peptide in accordance with aspect 6, wherein said trypsin protease is from an ectoparasite.
8. A peptide in accordance with any of the preceding aspects, wherein said peptide inhibits a trypsin protease protein from a Caligidae.
9. A peptide in accordance with aspect 8, wherein said Caligidae is selected from the group consisting of Pseudocaligus, Caligus and Lepeophtheirus.
10. A peptide in accordance with aspect 8, wherein said Caligidae is Lepeophtheirus salmonis.
11. A peptide in accordance with any of the preceding aspects, wherein said trypsin is *L. salmonis* trypsin-1 protein.
12. A pharmaceutical composition for use in the prevention and/or treatment of a disease or infection by inhibiting the activity of a trypsin proteinase, wherein said pharmaceutical composition comprises a peptide which inhibits a trypsin protease protein, wherein the peptide comprises an amino acid sequence selected from the group consisting of SEQ ID NO 3, SEQ ID NO 5 or SEQ ID NO 10, and variants thereof being at least 70% identical over the entire sequence with any of the sequences SEQ ID NO 3, SEQ ID NO 5 or SEQ ID NO 10.
13. A pharmaceutical composition in accordance with aspect12, wherein the disease or infection is caused by a parasite, and wherein the disease or infection is prevented or treated by inhibiting the activity of a trypsin proteinase.
14. A pharmaceutical composition in accordance with aspect 12 or 13, wherein said peptide inhibits a trypsin protease protein from a Caligidae.
15. A pharmaceutical composition in accordance with aspect 14, wherein said Caligidae is selected from the group consisting of Pseudocaligus, Caligus and Lepeophtheirus.
16. A pharmaceutical composition in accordance with aspect 14, wherein said Caligidae is salmon louse Lepeophtheirus salmonis.

## Claims

1. A peptide, wherein said peptide inhibits a trypsin protease protein of a Caligidae, wherein the peptide comprises an amino acid sequence selected from the group consisting of SEQ ID NO 4, SEQ ID NO 6, SEQ ID NO 7, SEQ ID NO 8 or SEQ ID NO 11 and variants thereof being at least 70% identical over the entire sequence with any of the sequences SEQ ID NO 4, SEQ ID NO 6, SEQ ID NO 7, SEQ ID NO 8 or SEQ ID NO 11.

2. A peptide, wherein said peptide inhibits a trypsin protease protein, wherein the peptide comprises an amino acid sequence selected from the group consisting SEQ ID NO 4, SEQ ID NO 65, SEQ ID NO 7, SEQ ID NO 8 or SEQ ID NO 11.

3. A peptide in accordance with claim 1, wherein the peptide comprises the amino acids of SEQ ID NO 4.

4. A peptide in accordance with claim 1, wherein the peptide comprises the amino acids of SEQ ID NO 6.

5. A peptide in accordance with claim 1, wherein the peptide comprises the amino acids of SEQ ID NO 7.

6. A peptide in accordance with claim 1, wherein the peptide comprises the amino acids of SEQ ID NO 8.

7. A peptide in accordance with claim 1, wherein the peptide comprises the amino acids of SEQ ID NO 10.

8. A peptide in accordance with claim 1, wherein the peptide inhibits a trypsin protease from a parasite.

9. A peptide in accordance with 8, wherein said trypsin protease is from an ectoparasite.

10. A peptide in accordance with any of the preceding claims, wherein said peptide inhibits a trypsin protease protein from a Caligidae.

11. A peptide in accordance with claim 10, wherein said Caligidae is selected from the group consisting of Pseudocaligus, Caligus and Lepeophtheirus.

12. A peptide in accordance with claim 10, wherein said Caligidae is Lepeophtheirus salmonis.

13. A peptide in accordance with any of the preceding claims, wherein said trypsin protease isLsTryp1 or rLsTryp1.

14. A pharmaceutical composition for use in the prevention and/or treatment of a disease or infection by inhibiting the activity of a trypsin proteinase, wherein said pharmaceutical composition comprises a peptide which inhibits a trypsin protease protein, wherein the peptide comprises an amino acid sequence selected from the group consisting of SEQ ID NO 4, SEQ ID NO 6, SEQ ID NO 7, SEQ ID NO 8 or SEQ ID NO, and variants thereof being at least 70% identical over the entire sequence with any of the sequences SEQ ID NO 4, SEQ ID NO 6, SEQ ID NO 7, SEQ ID NO 8 or SEQ ID NO.

15. A pharmaceutical composition in accordance with claim 14, wherein the disease or infection is caused by a parasite, and wherein the disease or infection is prevented or treated by inhibiting the activity of a trypsin proteinase.

16. A pharmaceutical composition in accordance with claim 14, wherein said peptide inhibits a trypsin protease protein from a Caligidae.

17. A pharmaceutical composition in accordance with claim 16, wherein said Caligidae is selected from the group consisting of Pseudocaligus, Caligus and Lepeophtheirus.

18. A pharmaceutical composition in accordance with claim 16, wherein said Caligidae is salmon louse Lepeophtheirus salmonis.

19. A pharmaceutical composition in accordance with claim 14, wherein said wherein said trypsin protease is LsTryp1 or rLsTryp1.
